# EUROPEAN PATENT APPLICATION

(11) **EP 2 241 555 A1**
(43) Date of publication of application: **20.10.2010**
(21) Application number: 09157855.9
(22) Date of filing: 14.04.2009
(51) Int. Cl.: C07D 239/47, C07D 239/48, C07D 401/12, C07D 403/12, A61K 31/506, A61P 9/04

(54) **New RAC1 inhibitors as potential pharmacological agents for heart failure treatment**

(71) Applicant: UNIVERSITA' DEGLI STUDI DI MILANO, 20122 Milano (IT); Chemilia AB, 141 83 Huddinge (SE); Pharmacelsus, 66123 Saarbrücken (DE); Universität Leipzig, 04109 Leipzig (DE); University of Tartu, 50090 Tartu (EE); Novamass, 90220 Oulu (FI); Università degli Studi di Genova, 16126 Genova (IT)
(72) Inventor: Ferri, Nicola, I-20133, Pozzuolo Martesana, MILANO (IT); CorsiniI, Alberto, I-20124, MILANO (IT); Ruggiero, Carmelina, I-16145, GENOVA (IT); Högberg, Marita, SE-14652, TULLINGE (SE); Tong, Weimin, SE-75264, UPPSALA (SE); Robitzki, Andrea Anneliese, DE-69519, VIERNHEIM (DE); Maran, Uko, EE-51003, TARTU (EE); Sild, Sulev, EE-51004, TARTU (EE); Garcia-Sosa, Alfonso Tlatoani, EE-51003, TARTU (EE); Batzl-Hartmann, Christine, DE-66133, SAARBRUECKEN (DE); Bartholomae, Pia, DE-66123, SAARBRUECKEN (DE); Uusitalo, Jouko Sakari, FI-90420, OULU (FI); Tolonen, Ari Tapio, FI-90120, OULU (FI); Rousu, Timo, FI-90580, OULU (FI); Hokkanen, Juho Kalevi, FI-90250, OULU (FI); Turpeinen, Miia, DE-70376, STUTTGART (DE)
(74) Representative: Long, Giorgio

(57) **Abstract**

The present invention finds application in the field of medicine and, in particular, to new compounds of formula (I) and pharmaceutical preparations containing them useful for the treatment and/or prevention of heart failure.

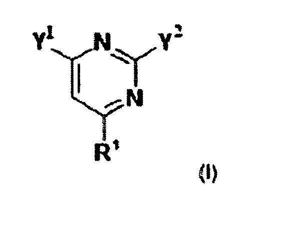

## Description

### DESCRIPTION OF THE INVENTION

The present invention finds application in the field of medicine and, in particular, to new compounds useful for the treatment and/or prevention of heart failure

### BACKGROUND

Heart failure constitutes one of the leading worldwide causes of morbidity and mortality Only, in Italy, it has been estimated that 1 million, which approximates 1.7 % of the population and about 22 million of people in the world, accounting for about 0.036% of the population, is affected by heart failure, Clinical monitoring has observed that 50% of people suffering from said pathology die within 5 years from the diagnosis. Thus, a large number of patients will benefit from the development of new pharmacological treatments. At present, pharmacological intervention includes the use of β-adrenergic receptor antagonists, inhibitors of angiotensin II, aldosterone and diuretics, which are currently employed in standard treatments for heart failure. Introduction of these pharmacological interventions have substantially increased the patient's survival and decreased morbidity However, these agents are far from ideal due to their side effects.

Accordingly, the identification of new molecular targets and the development of pharmacological agents for the treatment of heart failure represent an important scientific goal.

Recent *in vitro* and *in vivo* evidences and preliminary studies in clinic have indicated a pivotal role of Rac1 in the development of heart failure agents and, thus, it has become a leading target for the treatment of heart failure (Maack C, Kartes T, Kilter H, Schafers HJ, Nickenig G, Bohm M, Laufs U. Oxygen free radical release in human failing myocardium is associated with increased activity of rac1-GTPase and represents a target for statin treatment Circulation 2003;108:1567-1574; Sussman MA, Welch S, Walker A, Klevitsky R, Hewett TE, Price RL, Schaefer E, Yager K. Altered focal adhesion regulation correlates with cardiomyopathy in mice expressing constitutively active rac1. J Clin Invest 2000;105:875-886; Satoh M, Ogita H, Takeshita K, Mukai Y, Kwiatkowski DJ, Liao JK. Requirement of Rac1 in the development of cardiac hypertrophy Proc Natl Acad Sci U S A 2006;103:7432-7437).

In particular, Rac1 is a member of a RhoGTPase subfamily (Rac1-Rac4) that transduces extracellular signals from G-coupled protein receptors (GPCR), integrins and growth factor receptors to effector molecules that modulate multiple signalling pathways.

The Rho family comprises 22 genes encoding at least 25 proteins in humans; among them Rho, Rac, and Cdc42 proteins have been studied in the most detail. All Rho family members bind GTP (Guanosine-5'-triphosphate), which is an energy transfer molecule within the cells, and most exhibit GTPase activity and cycle between an inactive GDP-bound form and an active GTP-bound form. Said cycling activity is finely regulated by 3 groups of proteins: the guanine nucleotide exchange factors (GEFs) as activators, and the GTPase activating proteins (GAPs) and GDP dissociation inhibitors (GDIs) as negative regulators.

When bound to GTP, Rho GTPases interact with their downstream effectors, which include protein kinases, regulators of actin polymerization and other proteins with adaptive functions The selective interaction of the different Rho GTPases with a variety of effectors determines the final outcome For instance, the interaction of the Rho isoforms RhoA, RhoB, and/or RhoC with ROCK family kinases affects the actin organization, whereas their interaction with Dia1 stimulates actin polymerization The p21-activated kinase (PAK) family of proteins, on the other hand, act downstream of both Rac and Cdc42.

A classical approach to pharmacologically inhibit the Rho's activity is based on antagonizing the GTP binding to the GTP-binding domain of Rho proteins. However, high homology of the GTP binding domain among the Rho proteins hampers a specific pharmacological inhibition of a particular Rho protein This specificity of action is of particular interest since some of the biological actions of these proteins have opposite cellular effects thus causing undesired side effects.

Recently, a first-generation of specific inhibitor of Rac GTPase has been developed In particular, the chemical compound NSC23766 (see here below) 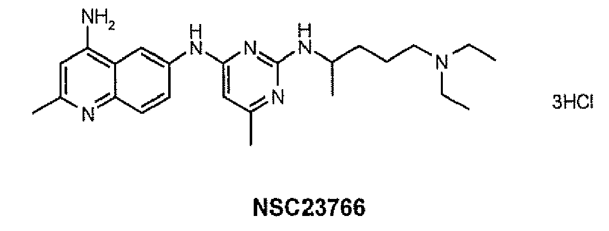 has been identified by a structure-based virtual screening of compounds fitting into a surface groove of Rac1 known to be critical for GEF specification. *In vitro,* it effectively inhibits Rac1 binding and activation by the Rac-specific GEF Trio or Tiam1 in a dose-dependent manner without interfering with the closely related Cdc42 or RhoA binding or activation by their respective GEFs NSC23766, interferes with the binding between Rac1 and GEF, thus directly affecting the exchange of GDP with GTP and the activation of its major effector PAK1.

However, relevant chemical modification of the structure of NSC23766 are necessary, in order to achieve a better fitting into the groove on the Rac1 surface, thus increasing the docking affinity, the inhibitory potency on Rac1 activity and obtaining more effective and specific drugs, which would show lower side effects.

### OBJECT OF THE INVENTION

Accordingly, it is a first object of the invention a compound as per claim 1 and dependent claims 2 to 3

In particular, the preferred compounds are those of claim 4, being the most preferred ones the compounds of claims 5 and 6.

As a second object, the compounds of the invention are used as a medicament, according to claim 7

In a preferred embodiment, said compounds may be used for the treatment or the prevention of human heart failure, cancers, hypertension, inflammation, and atherosclerosis, as per claim 8

As a further object, the present invention concerns a pharmaceutical composition comprising the compounds of the invention, as per claims 9-11.

### SUMMARY OF THE INVENTION

The present invention relates to new Rac1 inhibitors showing a higher inhibitory action compared to the prior-art compounds. The process for the chemical synthesis of said compounds, as well as the use of these compounds as potential drugs for the treatment of heart failure is disclosed as well.

### BRIEF DESCRIPTION OF THE FIGURES

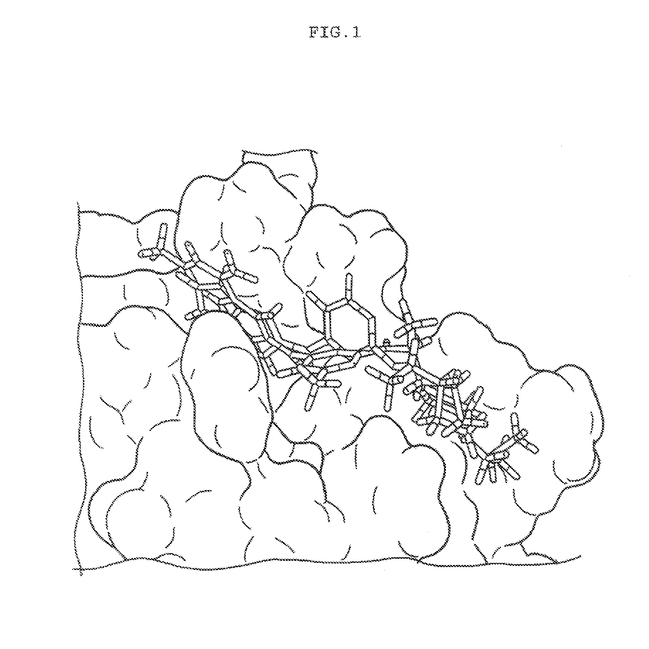

FIG. 1 shows the binding mode of complexes of Rac1 with CWB024 and the referenced compound NSC23766
FIG. 2 shows the results of the CWB024/02054 inhibition of the intracellular content of Rac1-GTP

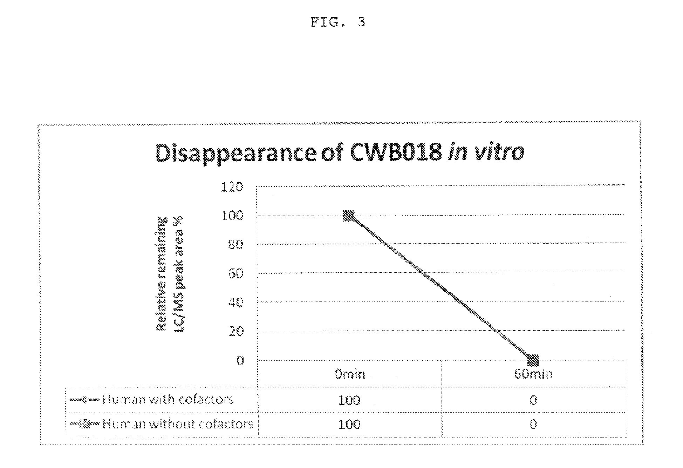

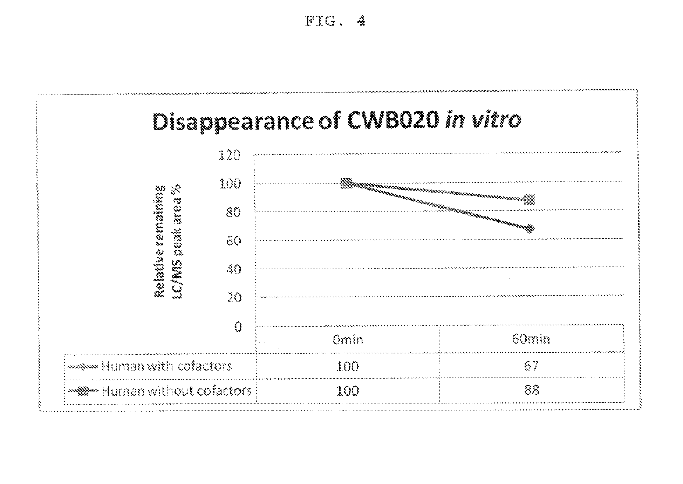

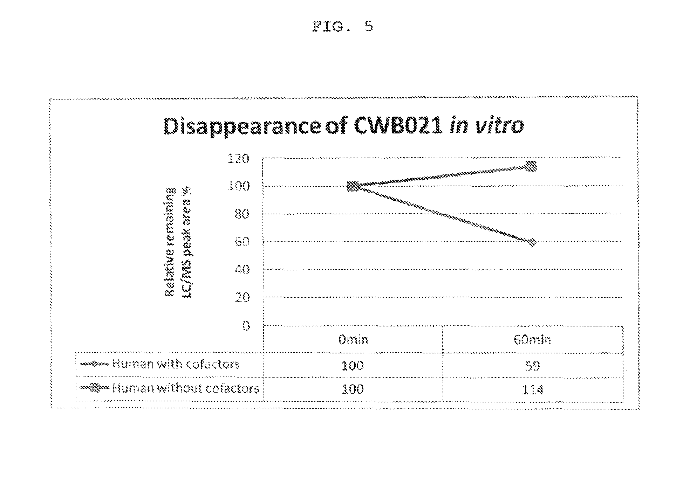

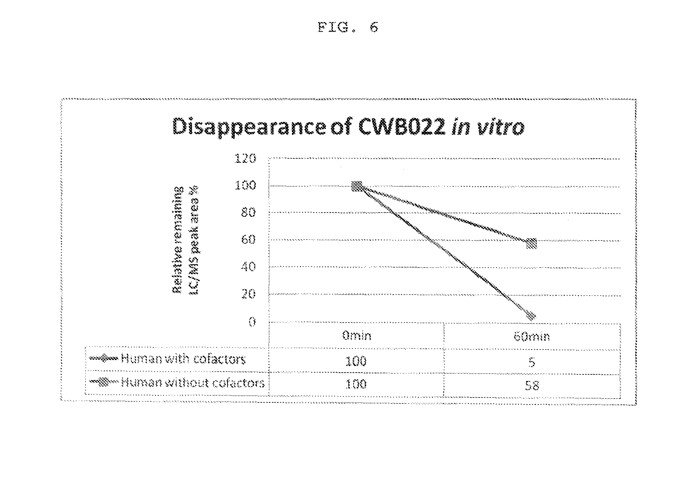

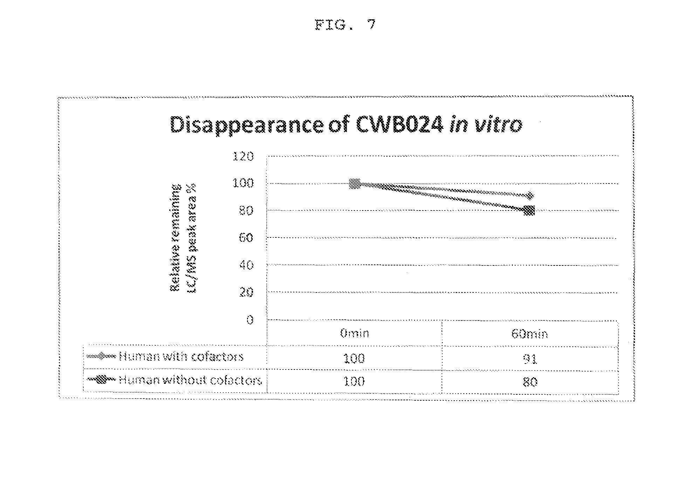

FIG. 3-7 show the relative remaining LC/MS peak areas of [M+H]^{+ 13}C -isotope peaks for CWB018, CWB020, CWB021, CWB022, CWB024 in 0 and 60 min incubations, with and without cofactors. Co-factors = NADPH, UDPGA, PAPS and GSH.

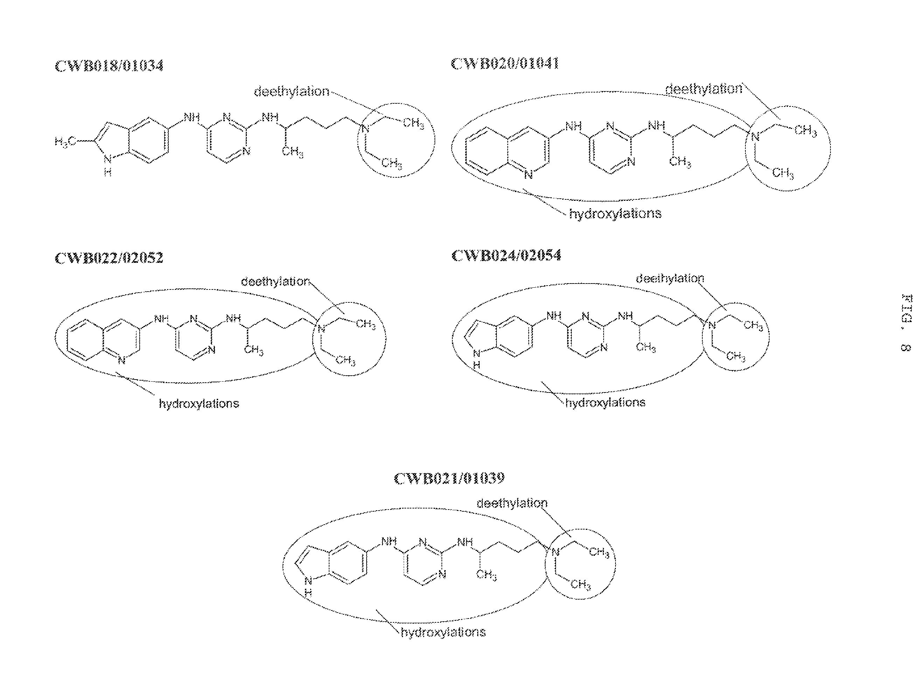

FIG 8 shows the metabolic reactions and metabolites detected for the invention compounds with Rac1 inhibitory activity

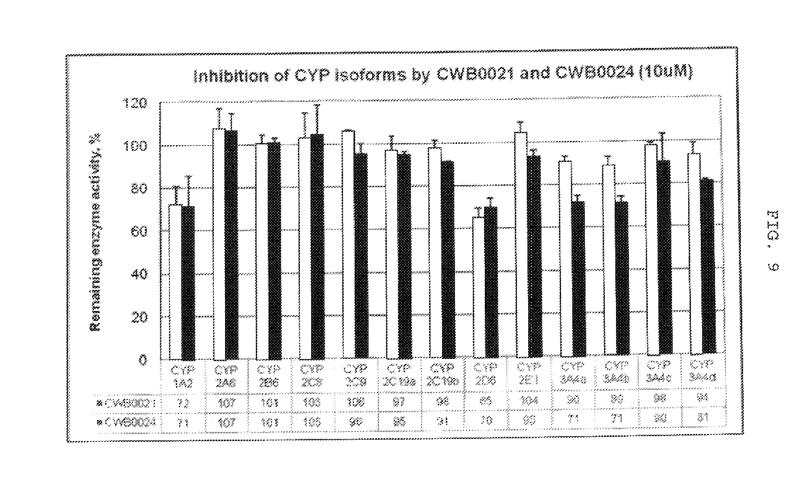

FIG. 9 which shows the relative remaining enzyme activities (%) with 10 µM CWB021/01039 (white) and 10 µM CWB024/02054 (black) in comparison to solvent control samples. The probe metabolites as in experimental For CYP2C19, a=omeprazole demethylation and b=omeprazole 5-hydroxylation; for CYP3A4, a=omeprazole 3-hydroxylation, b= omeprazole sulfonation, c=testosterone 6β-hydroxylation and d= midazolam 1'-hydroxylation.

### DETAILED DESCRIPTION OF THE INVENTION

According to the first embodiment, the present invention concerns compounds having the following general formula (I): 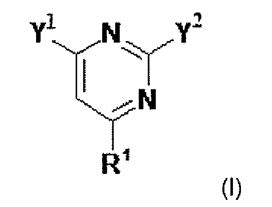 wherein
Y¹ may be
1) -NR²R³, wherein R² is H and R³ may be
   a) a saturated or unsaturated straight or branched C1-C18 carbon chain, optionally interrupted by one or more heteroatoms and being optionally substituted with one or more carboxylic acid (-COOH) or carboxylic acid ester (-COOR⁸) group or hydroxyl group (-OH) or thiol group (-SH) or halogen or amino group (-NH₂) or substituted amino group (-NMR⁸₂) or nitro group (-NO₂) or with aromatic group Ar having from 9 to 10 ring carbons, wherein from 1 to 4 carbon atoms may be substituted with a heteroatom selected from -N-, -O- and -S-, said Ar group being optionally substituted at any available position with a saturated or unsaturated straight or branched C1-C4 carbon chain or a straight or branched C1-C6 alkoxy group or C3-C6 cycloalkyl group or a hydroxy group (-OH) or a thiol group (-SH) or an halogen or an amino group (-NH₂) or a substituted amino group (-NR⁸₂) or a nitro group (-NO₂), wherein R⁸ is a C1-C8 straight or branched alkyl group, or
   b) R⁴, wherein R⁴ is an aromatic group Ar as above disclosed; or wherein
   c) R² and R³, together with the N atom they are attached to, form a 5 or 6 membered heterocycle, wherein from 1 to 3 carbon atoms may be substituted with an heteroatom selected from -N-, -O- or -S-, said heterocycle being optionally substituted at any available position with a saturated or unsaturated straight or branched C1-C4 carbon chain or a C3-C6 cycloalkyl group or a straight or branched C1-C6 alkoxy group or with one or more carboxylic acid (-COOH) or carboxylic acid ester (-COOR⁸) group or a hydroxy group (-OH) or a thiol group (-SH) or an halogen or an amino group (-NH₂) or a substituted amino group(-NR⁸₂), wherein R⁸ is as above disclosed, or a nitro group (-NO₂),or wherein Y1 may be
2) -O-R³, wherein R³ is as above disclosed, and wherein
   Y² is -N-R⁵R⁶, wherein
   d) R⁵ is H and R⁶ is R³ or R⁴, wherein R³ and R⁴ are as above disclosed; or wherein
   e) R⁵ and R⁶ together with the N atom they are attached to form a 5 or 6 membered heterocycle, wherein from 1 to 3 carbon atoms may be substituted with an heteroatom selected from -N-, -O- or -S-, said heterocycle being optionally substituted at any available position with a saturated or unsaturated straight or branched C1-C4 carbon chain or a C3-C6 cycloalkyl group or a straight or branched C1-C6 alkoxy group or with one or more carboxylic acid (-COOH) or carboxylic acid ester (-COOR⁸) group or a hydroxy group (-OH) or a thiol group (-SH) or an halogen or an amino group (-NH₂) or a substituted amino group(-NR⁸₂), wherein R⁸ is as above disclosed, or a nitro group (-NO₂); and wherein
      R¹ is H or a saturated or unsaturated straight or branched C1-C18 carbon chain; or any salts thereof.

Preferably, the compounds of the invention are those of formula (I) above wherein
1) when Y¹ is NR²R³, R² is H and R³ is
   a) a saturated straight or branched C1-C7 carbon chain optionally substituted with one or more carboxylic acid (-COOH) group or carboxylic acid ester (-COOR⁸) group or a substituted amino group (-NR⁸₂) or with an aromatic group Ar having from 9 to 10 ring carbons, wherein one carbon atom is replaced with nitrogen, said Ar group being optionally substituted at any available position with one or more (an) hydroxyl (-OH) group or methyl group (-CH₃), wherein R⁸ is a C1-C4 saturated straight or branched carbon chain; or
   b) R³ is R⁴, wherein R⁴ is an Ar group having from 9 to 10 ring carbons, wherein one carbon atom is replaced with nitrogen, said Ar group being optionally substituted at any available position with one or more hydroxy (-OH) group or methyl group (-CH₃), or
   c) R² and R³ together with the nitrogen atom they are attached to form a 6 membered heterocycle optionally comprising an oxygen atom;
   or wherein
2) Y¹ may be -O-R⁴, wherein R⁴ is an aromatic group Ar having 10 ring carbons, wherein optionally one carbon atom may be substituted with nitrogen, said Ar group being optionally substituted at any available position with a methyl group (-CH₃);
and wherein Y² is -NR⁵R⁶, wherein
d) R⁵ is H and R⁶ is a saturated straight or branched C1-C7 carbon chain optionally substituted with a substituted amino group (-NR⁸₂) or with an aromatic group Ar having 9 ring carbons, wherein one carbon atom is replaced with nitrogen, said Ar group being optionally substituted at any available position with one or more an hydroxyl (-OH) group and wherein R⁸ is as above disclosed; or wherein
e) R⁵ and R⁶ together with the nitrogen atom they are attached to form a 6 membered heterocycle, wherein from 1 to 2 carbon atoms are substituted with nitrogen or oxygen; and wherein R1 is H or C1-C4 straight or branched saturated carbon chain; or any salts thereof.

More preferably, the compounds of the invention are those of formula (I) above wherein when Y¹ is NR²R³ and R² is H, R³ is
a) -(CH)(COOCH₃)(CH₂-CH-(CH₃)₂) or -CH(CH₃)(CH₂)₃-N(CH₂CH₃)₂ or -CH₂-COOH or -CH₂-COOCH₂CH₃; or a - CH₂- or a -CH₂-CH₂- group substituted with indole substituted with a hydroxyl (-OH) or methyl (-CH₃) group;
b) quinoline or isoquinoline or indole, optionally substituted with a hydroxyl (-OH) or methyl (-CH₃) group or both, or
c) R² and R³ together with the nitrogen atom they are attached to form a piperidine or a morpholine cycle;
   or when Y¹ is -O-R³, wherein R³ is quinoline or isoquinoline or naphthalene, optionally substituted with a hydroxyl (-OH) or methyl (-CH₃) group;
   and (wherein) when Y² is -NR⁵R⁶, wherein R⁵ is H and R⁶ may be
d) -CH(CH₃)(CH₂)₃-N(CH₂CH₃)₂ or -CH-(CH₂)₃-N(CH₂CH₃)₂ or -CH-(CH₂)₄-N(CH₂CH₃)₂ or a -CH₂- or a -CH₂-CH₂- group substituted with indole substituted with a hydroxyl (-OH) or methyl (-CH₃)group or R⁶ may be
   quinoline or isoquinoline, optionally substituted with a hydroxyl (-OH) or methyl (-CH₃) group or both; or
e) R⁵ and R⁶ together with the nitrogen atom they are attached to form a piperidine or morpholine ring,
   and wherein R¹ is H or methyl; or any salts thereof

Even more preferably, the compounds of the invention are:
N2-(4-Diethylamino-1-methyl-butyl)-N4-quinolin-6-yl-pyrimidine-2,4-diamine;
N1,N1-Diethyl-N4-(4-methyl-6-morpholin-4-ylpyrimidin-2-yl)-pentane-1,4-diamine;
N1,N1-Diethyl-N4-(6-methyl-2-morpholin-4-yl-pyrimidin-4-yl)-pentane-1,4-diamine;
[2-(4-Diethylamino-1-methyl-butylamino)-6-methyl-pyrimidin-4-ylamino]-acetic acid ethyl ester;
4-Methyl-2-[6-methyl-2-(quinolin-6-ylamino)-pyrimidin-4-ylamino]-pentanoic acid methyl ester;
N1,N1-Diethyl-N4-[4-naphthalen-2-yloxy)-pyrimidin-2-yl]-pentane-1,4-diamine; 3-{2-[2-(4-Diethylamino-1-methyl-butylamino)-6-methyl-pyrimidin-4-ylamino]-ethyl}-1 H-indol-5-ol;
3-{2-[4-(4-diethylamino-1-methyl-butylamino)-6-methyl-pyrimidin-2-ylamino]-ethyl}-1H-indol-5-ol;
N1,N1-Diethyl-N4-[4-methyl-6-(2-methyl-quinalin-6-yloxy)-pyrimidin-2-yl]-pentane-1,4-diamine;
N2-(4-Diethylamino-1-methyl-butyl)-N4-[2-(1H-indol-3-yl)-ethyl)-6-methylpyrimidine-2,4-diamine,
N2-(4-Diethylamino-1-methyl-butyl)-6-methyl-N4-quinolin-6-yl-pyrimidine-2,4-diamine;
6-[2-(4-Diethylamino-1-methyl-butylamino)-6-methyl-pyrimidin-4-ylamino]-2-methyl-quinolin-4-ol;
6-{2-[2-(1H-Indol-3-yl)-ethylamino]-6-methyl-pyrimidin-4-ylamino}-2-methyl-quinolin-4-ol;
N1,N1-Diethyl-N4-(4-methyl-8-piperidin-1-yl-pyrimidin-2-yl)-pentane-1,4-diamine; N,N-Diethyl-N'-[4-methyl-6-(2-methyl-quinolin-6-yloxy)-pyrimidin-2-yl]-propane-1,3-diamine;
[2-(4-Diethylamino-1-methyl-butylamino)-6-methyl-pyrimidin-4-ylamino]-acetic acid;
N2,N4-Bis-(1H-indol-5-ylmethyl)-pyrimidine-2,4-diamine,
N2-(4-Diethylamino-1-methyl-butyl)-N4-(2-methyl-1H-indol-5-yl)-pyrimidine-2,4-diamine;
N2-(4-Diethylamino-1-methyl-butyl)-N4-quinolin-3-yl-pyrimidine-2,4-diamine;
N2-(4-Diethylamino-1-methyl-butyl)-N4-(1H-indol-6-yl)-pyrimidine-2,4-diamine;
N2-(4-Diethylamino-1-methyl-butyl)-N4-(1H-indol-5-yl)-pyrimidine-2,4-diamine;
3-{2-[2-(4-Diethylamino-butylamino)-6-methyl-pyrimidin-4-ylamino]-ethyl)-1H-indol-5-ol, or any pharmaceutically acceptable salts thereof

In a further embodiment, the preferred compounds are 4-Methyl-2-[6-methyl-2-(quinolin-6-ylamino)-pyrimidin-4-ylamino]-pentanoic acid methyl ester, N2,N4-Bis-(1H-indol-5-ylmethyl)-pyrimidine-2,4-diamine, N2-(4-Diethylamino-1-methyl-butyl)-N4-(2-methyl-1H-indol-5-yi)-pyrimidine-2,4-diamine, N2-(4-Diethylamino-1-methylbutyl)-N4-quinolin-3-yl-pyrimidine-2,4-diamine, N2-(4-Diethylamino-1-methyl-butyl)-N4-(1H-indol-5-yl)-pyrimidine-2,4-diamine, or any pharmaceutically acceptable salts thereof, being N2-(4-Diethylamino-1-methyl-butyl)-N4-(1H-indol-5-yl)-pyrimidine-2,4-diamine or any pharmaceutically acceptable salt thereof the most preferred one. Within the present invention a "saturated carbon chain" relates to straight or branched alkylic chains, i.e. comprising carbon atoms linked by single bonds Preferred saturated carbon chains comprise from 1 to 18 carbon atoms. More preferable, the saturated carbon chain comprises from 1 to 4 carbon atoms, such as, methyl (-CH₃), ethyl (-CH₂-CH₃), propyl (-CH₂-CH₂-CH₃), iso-propyl (-CH(CH₃)CH₃),
n-butyl (-CH₂-CH₂-CH₂-CH₃), iso-butyl (-CH₂(CH₂)CH₂-CH₃)or ter-butyl ((-CH₂-CH(CH₃)(CH₃))

"Unsaturated carbon chain" refers to a straight or branched carbon chain comprising one or more double or triple bonds Preferred unsaturated carbon chains comprise from 2 to 18 carbon atoms

More preferably, unsaturated carbon chain comprise from 2 to 8 carbon atoms.

"Alkoxy group" refers to saturated or unsaturated O-carbon chains comprising from 1 to 18 carbon atoms. Preferred alkoxy group comprise from 1 to X carbon atoms, such as for instance, methoxy, ethoxy, n-propoxy, isopropoxy, n-butoxy, isobutoxy, sec-butoxy and tert-butoxy, n-pentoxy, n-hexoxy, isohexoxy, n-heptoxy, isoheptoxy. "Cyclic group" refers to saturated carboncycles of from 3 to 8 carbon atoms. Preferred cyclic group have from 3 to 6 carbon atoms. More preferably, they include, cyclopropane, cyclobutane, cyclopentane and cyclohexane.

"Heteroatom" refers to any one of nitrogen, oxygen and sulphur "Aromatic cycle", also referred to as Ar, includes any aromatic monocyclic or bicyclic or even tricyclic groups. Ar groups may comprise 5 or more carbon atoms Preferred aromatic cycles are, for instance, benzene, naphthalene.

"Heteroeyclic group" or "heterocycles" includes any saturated cyclic group of from 3 or more carbon atoms, wherein at least one and up to 3 carbon atoms are substituted with an heteroatom.

Heterocycles include, for instance, Tetrahydrofuran, tetrahydrothiophene, pyrrolidine, 1,2,3-triazolidine, tetrahydropyran, piperidine, 1,4-dioxane, morpholine and thiazolidine Preferred heterocyclic rings are, for instance, 5 and 6 membered rings comprising at least one nitrogen atom. More preferably, said heterocyclic rings are morpholine and piperidine

"Heteroaromatic group" also includes not completely unsaturated heterocyclic groups, such as, for instance, dihydropyran, imidazoline, pyrrolidine, 2H- and 4H-pyran.

"Heteroaromatic ring" refers to a cyclic aromatic group, wherein one or more carbon atom has been substituted with an heteroatom

Preferred heteroaromatic groups within the present invention include furan, thiazole, thiophene, pyrrole, isoxazole, pyrazine, oxazole, pyrazole, imidazole, 1,2,3- and 1,2,4 -triazole, 1,2,3-, 1,2,4- and 1,2,5-oxadiazole, pyridine, pyrimidine, pyridazine, 1,3,5-, 1,2,3- and 1,2,4-triazine

Aromatic and heteroaromatic rings may additionally be benzo-fused and substituted at any available positions, i e at those atoms which are not engaged in fusion or which are not already substituted

For instance, naphtalene is the benzofused ring of benzene, indole is the benzofused ring of pyrrole, benzothiophene is the benzo-fused ring of thiophene, benzofuran of furan, benzoxazole of oxazole, benzimidazole of imidazole, quinoline and isoquinoline of pyridine

"Halogen" includes fluorine and iodine, but especially bromine and in particular chlorine.

"Amino acid residue" include any natural or non-natural α-amino acids, such as, for instance, glycine, alanine, cysteine, lysine, valine, proline, leucine, arginine, threonine, tryptophan, phenylalanine, tyrosine, glutamic acid, asparagine, aspartic acid, glutamine.

Preferred amino acids within the present invention are glycine, alanine, serine, proline, lysine, valine, treonine, cysteine, leucine, isoleucine and methionine More preferably, the amino acid is glycine (-NH-CH₂-COOH).

"Amino acid residue derivative" includes any derivatives of amino acid, such as, for instance, obtained by modifications occurring at the carboxy and/or at the amino terminal For instance, the carboxy group may be esterified according to known methods to give the alkyl derivative, such as, for instance, the methyl or the ethyl ester. Conversely, the amino group may be suitably acylated by conventional ways, to give the acetyl derivative,

A preferred modification includes the esterification of the carboxy-terminal or the amino acid. Preferably, said esterification leads to the methyl ester of the amino acid.

Within the present invention, a preferred amino acid residue derivative is glycine methyl ester

As for the preparation of the compounds of the invention, they can be synthesized according to Scheme 1 and Scheme 2 below.

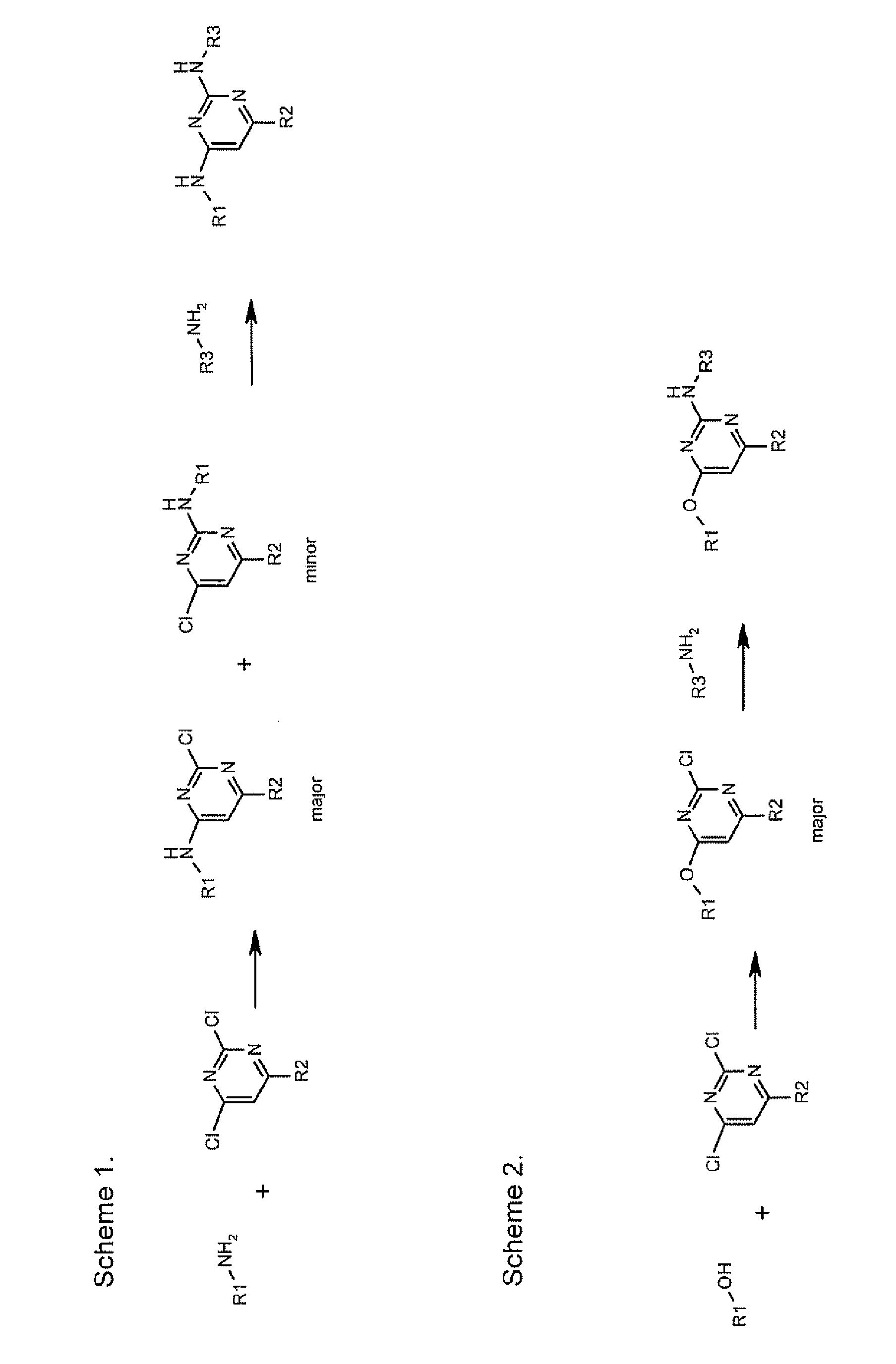

Reactions in Scheme 1 are reactions between amines R1-NH₂, R3-NH₂ and 2,4-dichloropyrimidine which are amination reactions onto a haloaromatic ring The major product after the first step is the amination product on 4-position of 2,4-dichloropyrimidine.

The first step in Scheme 2 is aroxylation of haloaromatic ring, the reaction between aromatic alcohol and 2,4-dichloropyrimidine. The second step is the same amination reaction onto a haloaromatic ring as in Scheme 1

The synthesis of the specific compounds is given in the following Examples 1 to 20. The present invention also concerns a pharmaceutical preparation comprising one or more of the compounds of the invention

In particular, said compounds are in the form of a salt and, preferably, in the form of a pharmaceutically acceptable salt.

"Pharmaceutically acceptable salt" is intended to include any salts suitable to be administered to human or animal and having suitable technological properties, such as, for instance, sodium, potassium, ammonium, zinc salt or any salts with amino acids, such as with lysine (see, for a general reference, Remington's Pharmaceutical Sciences Handbook, Mack Pub Co., N.Y., USA 17^{th} edition, 1985)

The pharmaceutical preparations of the invention comprise in addition to one or more of the compounds of the invention, and according to the type of formulation to be used, other pharmaceutical additives and/or excipients conventional in the art, such as, for instance, diluents, solvents, bulking agents, fillers, reological modifiers, stabilizers, binders, lubricants, disintegrants, preservatives, pH adjusting agents, buffers, antioxidants, chelating agents, plasticizers, polymers, emulsifiers, edulcorants, flavoring agents, etc., alone or in combination.

### Example 1

### N2-(4-Diethylamino-1-methyl-butyl)-N4-uinolin-6-pyrimidine-2,4-diamine (CWB004)

The mixture of 6-aminoquinoline (200 mg, 1.39 mmol), 2,4-dichloropyrimidine (249 mg, 1.67 mmol) and diisopropylethylamine (274 mg, 2.09 mmol) in isopropanol (4 mL) was stirred in a pressure bottle at 120 °C for 19 h. The reaction mixture was evaporated under vacuum and the residue was purified by silica gel column using EtOAc/hepfiane 2/8, 1/1 and pure EtOAc as eluent to give 200 mg (56 %) of 6-(2-chloro-pyrimidin-4-ylamino)quinoline.

The mixture of 6-(2-chloro-pyrimidin-4-ylamino)quinoline (200 mg, 0779 mmol) and 2-amino-5-diethylaminopentane (148 mg, 0 935 mmol) in ethylene glycol (4 mL) was stirred in a pressure bottle at 110 °C for 2 days. Saturated NaHCO₃ solution was added to the reaction mixture and it was extracted with EtOAc, the organic layer was dried over Na₂SO₄, filtrated and evaporated under vacuum The residue was purified by silica gel column using EtOAc, 10 % MeOH in EtOAc and EtOAc/MeOH/Et₃N 7:3:0.5 as eluent to give 200 mg (68 %) of the titled compound.

¹H NMR (300 MHz, CDCl₃). δ 8.83 (dd, 1H), 8.10-7.99 (m, 4H), 7.66 (dd, 1H), 738 (dd, 1H), 6.81 (s, 1H), 6.08 (d, 1H), 4.96 (d, 1 H), 4.11 (m, H), 259-244 (m, 6H), 1.61-1.57 (m, 4H), 1.28 (d, 3H), 1.07-1.00(m, 6H)

### Example 2

### N1,N1-Diethyl-N4-(4-methyl-6-morpholin-4-yl-pyrimidin-2-yl)-pentane-1,4-diamine (CWB007) and N1,N1-Diethyl-N4-(6-methyl-2-morpholin-4-yl-pyrimidin-4-yl)-pentane-1,4-diamine (CWB016)

The mixture of 2-amino-5-diethyl-aminopentane (1.9 g, 12.0 mmol), 2,4-dichloro-6-methylpyrimidine (1.63 g, 10.0 mmol) and diisopropylethylamine (1.93 g, 15.0 mmol) in isopropanol (20 mL) was stirred in a pressure bottle at 100 °C overnight The reaction mixture was evaporated under vacuum and the residue was purified by silica gel column using EtOAc/MeOH/Et₃N 7:3:0 3 as eluent to give 760 mg (27 %) of N1,N1-diethyl-N4-(4-chloro-6-methyl-pyrimidin-2-yl)-4-methylpentane-1,4-diamine and the mixture of N1,N1-diethyl-N4-(4-chloro-6-methyl-pyrimidin-2-yl)-4-methylpentane-1,4-diamine and N1,N1-diethyl-N4-(2-chloro-6-methyl-pyrimidin-4-yl)-4-methylpentane-1,4-diamine

The mixture of N1,N1-diethyl-N4-(4-chioro-6-methy)-pyrimidin-2-yl)-4-methylpentane-1,4-diamine and N1,N1-diethyl-N4-(2-chloro-6-methyl-pyrimidin-4-yl)-4-methyfpentane-1,4-diamine (200 mg, 070 mmol), morpholine (179 mg, 2.10 mmol) and diisopropylethylamine (275 mg, 2.10 mmol) in butanol (5 mL) was stirred in a pressure bottle at 150 °C overnight. The reaction mixture was evaporated under vacuum and the residue was purified by silica gel column using EtOAc/MeOH/Et₃N 7.3.0.3 as eluent to give 70 mg (30 %) of the first title compound (CWB007) and 25 mg (8.4 %) of the second title compound (CWB016). Both compounds were dissolved in CH₂Cl₂/diethyfether and 10 mL of 1M HCl in diethylether was added The solvent was evaporated to give HCl salts of above compounds.
¹H NMR (300 MHz, CDCl₃)_{.} δ 5.71 (s, 1H), 4 65 (d, 1H), 4 03 (m, 1H), 3.75 (t, 4H), 3.53 (t, 4H), 2.51 (q, 4H), 2.43 (m, 2H), 2.19 (s, 3H), 1.51 (m, 4H), 1 18 (d, 3H), 1.00 (t, 6H) (CWB007)
¹H NMR (300 MHz, CDCl₃): δ 5.54 (s, 1H), 4.69 (d, 1 H), 372-3.83 (m, 9H), 2 52 (q, 4H), 2 42 (m, 2H), 2.19 (s, 3H), 1.51 (m, 4H), 1.18 (d, 3H), 1.01 (t, 6H) (CWB016)

### Example 3

### [2-(4-Diethylamino-1-methyl-butylamino)-6-methyl-pyrimidin-4-ylamino]-acetic acid ethyl ester (CWB012)

The mixture of glycine ethyl ester HCl (2.0 g, 14 3 mmol), 2,4-dichloro-6-methylpyrimidine (2.66 g, 15.7 mmol) and diisopropylethylamine (4.08 g, 31.5 mmol) in isopropanol (10 mL) was stirred in a pressure bottle at 120 °C for 24 h. The reaction mixture was evaporated under vacuum and the residue was purified by silica gel column using EtOAc/heptane 2/8, 1/1 and pure EtOAc as eluent to give 1.93 g (59 %) of (2-chloro-6-methyl-pyrimidin-4-yiamino)-acetic acid ethyl ester.

The mixture of (2-chloro-6-methyl-pyrimidin-4-ylamino)-acetic acid ethyl ester (500 mg, 2 18 mmol) and 2-amino-5-diethylaminopentane (399 mg, 2 62 mmol) was stirred in a pressure bottle at 150 °C for 2 h. CH₂Cl₂ was added to the mixture and it was washed with saturated NaHCO₃ and H₂O, The organic layer was separated, dried under Na₂SO₄ and evaporated under vacuum The residue was purified by silica gel column using EtOAc, 10 % MeOH in EtOAc and EtOAc/MeOH/Et₃N 7:3:0.3 as eluent to give 210 mg (27 %) of the titled compound The compound was made to its HCl salt using the same procedure as in Example 2.
¹H NMR (300 MHz, CDCl₃). δ 5.61 (s, 1H), 4.98 (br s, 1H), 4.67 (d, 1H), 4.23 (q, 2H), 4.11 (t, 2H), 4,07-4.00 (m, 1H), 2.54 (q, 4H), 2.46 (t, 2H), 2.16 (s, 3H), 1.53-1.45 (m, 4H), 1.29 (t, 3H), 1 16 (d, 3H), 1.03 (t, 6H).

### Example 4

### 4-Methyl-2-[6-methyl-2-(quinolin-6-ylamino)-pyrimidin-4-ylamino]-pentanoic acid methyl ester (CWB018)

The title compound was prepared from L-leucine methyl ester as described in Example 3. 6-aminoquinoline was used instead of 2-amino-5-diethylaminopentane
¹H NMR (300 MHz, CDCl₃): δ 8.76 (dd, 1H), 8.39 (d, 1H), 8.14 (d, 1H), 8.00 (d, 1H), 7.65 (dd, 1H), 7 35 (dd, 1H), 7 14 (s, 1H), 5.87 (s, 1H), 4.96 (d, 1H), 4 79 (br. s, 1H), 3 68 (s, 3H), 2.28 (s, 3H), 1.82-1.67 (m, 3H), 0.99 (dd, 6H).

### Example 5

### N1,N1-Diethyl-N4-[4-nahthalen-2-yloxy)-pyrimidin-2-yl]-pentane-1,4-diamine (CWB019)

2-Naphthol (1.0 g, 6 94 mmol) was dissolved in THF/DMF 1:1 (30 mL) and NaH (278 mg, 6 94 mmol) was added in portions The mixture was stirred under room temperature for 45 min. 2,4-dichloropyrimidine (1.034 g, 6.94 mmol) was added to the mixture and it was stirred at same temperature overnight H₂O (60 mL) was added to the reaction mixture and the formed precipitate was filtrated and then coevaporated with toluene to give 1.655 g (92 %) of 2-(2-chloro-pyrimidin-4-yloxy)-napthalene.

2-(2-chloro-pyrimidin-4-yloxy)-napthalene (0 50 g, 1 95 mmol) and 2-amino-5-diethylaminopentane (0.618 g, 3.9 mmol) was stirred in a pressure bottle at 150 °C for 1.5 h. CH₂Cl₂ was added to the mixture and it was washed with saturated NaHCO₃ and H₂O The organic layer was separated and evaporated under vacuum The residue was purified by silica gel column using EtOAc, 10 % MeOH in EtOAc and EtOAc/MeOH/Et₃N 7:3:0 5 as eluent to give 0.39 g (53 %) of the title compound. The compound was made to its HCl salt using the same procedure as in Example 2.
¹H NMR (300 MHz, CDCl₃): δ 8.12 (d, 1H), 7.87-7.76 (m, 3H), 7.58 (d, 1H), 7.50-7.46 (m, 2H), 7.28 (dd, 1H), 6.06 (d, 1H), 5.13 (d, 1H), 3.90 (br. s, 1H), 2.44 (q, 4H), 2.34 (br. s, 2H), 1 44 (br. s, 2H), 1.13 (d, 3H), 0.95 (t, 6H).

### Example 6

### 3-{2-[2-(4-Diethylamino-1-methyl-butylamino)-6-methyl-pyrimidin-4-ylamino]-ethyl}-1H-indol-5-ol (CWB025) and 3-{2-[4-(4-diethylamino-1-methyl-butylamino)-6-methyl-pyrimidin-2-ylamino]-ethyl}-1H-indol-5-ol (CWB027)

The mixture of serotonin hydrochloride (1.0 g, 4.72 mmol), 2,4-dichloro-6-methylpyrimidine (1.0 g, 6.13 mmol) and diisopropylethylamine (1.0 g, 100 mmol) in isopropanol (8 mL) was stirred in a pressure bottle at 120 °C overnight The reaction mixture was evaporated under vacuum and the residue was purified by silica gel column using CH₂Cl₂/MeOH as eluent to give 500 mg (50 %) of 3-(2-chloro-6-methyl-pyrimidin-4-ylamino)-1H-indol-5-ol and 200 mg (14 %) of 3-(4-chloro-6-methyl-pyrimidin-2-ylamino)-,1H-indol-5-ol

The first title compound was prepared from 3-(2-chloro-6-methyl-pyrimidin-4-ylamino)-1H-indol-5-ol as described in Example 3.
¹H-NMR (300 MHz, CDCl₃ + CD₃OD): δ 7.16(d, 1H), 6.94-6.96 (m, 2H), 6.76 (dd, 1H), 5.47 (s, 1H), 4.01 (m, 1H), 3.50 (m 2H), 2 92 (t, 2H), 2.47-2.61 (m, 6H), 2.10 (s, 3H), 1.44-1.67 (m, 4H), 1.13 (d, 3H), 0.98-1.09 (m, 6H) (CWB025)

The second title compound was prepared from 3-(4-chloro-6-methyl-pyrimidin-2-ylamino)-1H-indol-5-ol as described in Example 3.
¹H-NMR (300 MHz, CDCl₃): δ 8.21 (br, 1H), 7.11 (d, 1H), 6.98(d, 1H), 6.87 (s, 1H), 6.75 (dd, 1H), 5.50 (s, 1H), 5.08 (br, 1H), 4.67 (br, 1H), 4.01 (br, 1H), 3.67 (m, 1H), 3.57 (m, 1H), 2.57 (t, 2H), 2.47-2.1 (m, 6H), 2.15 (s, 3H), 1.49-1.57 (m, 4H), 1.13 (d, 3H), 1.02 (t, 6H) (CWB027)

### Example 7

### N1,N1-Diethyl-N4-[4-methyl-6-(2-methyl-quinolin-6-yloxy)-pyrimidin-2-yl]-pentane-1,4-diamine (CWB001)

The title compound was prepared from 6-hydroxy-2-methylquinoline as described in Example 5.
¹H NMR (300 MHz, CDCl₃). δ 8.04-7.99 (m, 2H), 7.50-7.47 (m, 2H), 7.31 (d, 1H), 5.91 (s, 1H), 4.94 (d, 1H), 3.88 (br s, 1H), 2 76 (s, 3H), 2.45 (q, 4H), 2.33 (br s, 2H), 2.27 (s, 3H), 1 64 (br s, 2H), 1.42 (br. s, 2H), 1 10 (d, 3H), 0 96 (t, 6H).

### Example 8

### N2-(4-Diethylamino-1-methyl-butyl)-N4-[2-(1H-indol-3-yl)-ethyl)-6-methylpyrimidine-2,4-diamine (CWB002)

The title compound was prepared from tryptamine hydrochloride as described in Example 3
¹H-NMR (300 MHz, CDCl₃). δ 8.55(br, 1H), 7 60 (d, 1H), 7 37 (d, 1H), 7.17 ddd, 1H), 7.10 (ddd, 1H), 7.03 (d, 1H), 5 51 (s, 1H) 4.63 (br, 2H), 4.06 (m, 1H), 3.62 (m, 2H), 3.05 (t, 2H), 2.42-2.57 (m, 6H), 2.15 (s, 3H), 1.43-1.57 (m, 4H), 1.17 (d, 3H), 1.01 (t, 6H).

### Example 9

### N2-(4-Diethylamino-1-methyl-butyl)-6-methyl-N4-quinolin-6-yl-pyrimidine-2,4-diamine (CWB003)

The title compound was prepared from 6-aminoquinoline as described in Example 3
¹H NMR (300 MHz, CDCl₃): δ 8.83 (dd,1H), 8.09-8.05 (m, 3H), 7.66 (dd, 1H), 7 39 (dd, 1H), 6.74 (s, 1H), 5.97 (s, 1H), 4.90 (d, 1H), 4 12 (m, 1H), 2.67-2.47 (m, 6H), 1.60-1.58 (m, 4H), 1 28 (d, 3H), 1.04-0.99 (m, 6H)

### Example 10

### 6-[2-(4-Diethylamino-1-methyl-butylamino)-6-methyl-pyrimidin-4-ylamino]-2-methyl-quinolin-4-ol (CWB005)

The title compound was prepared from 6-amino-4-hydroxy-2-methylquinoline as described in Example 3.
¹H NMR (300 MHz, CDCl₃): δ 8.26 (s, 1H), 7.42-7.83 (m, 4H), 6.08 (s, 1H), 5.88 (s, 1H), 4.84 (m, 1H), 4 07 (m 1H), 2.36-2.52 (m, 9H), 2.12 (s, 3H), 1.49 (m, 4H), 1 14 (d, 3H), 0.97 (t, 6H).

### Example 11

### 6-{2-[2-(1H-Indol-3-yl)-ethylamino]-6-methyl-pyrimidin-4-ylamino}-2-methyl-quinolin-4-ol (CWB006)

The title compound was prepared from 6-amino-4-hydroxy-2-methylquinoline as described in Example 3 Tryptamine hydrochloride was used instead of 2-amino-5-diethylaminopentane.
¹H-NMR (300 MHz, CDCl₃ + CD₃OD), δ 8.25 (m, 1H), 7.99 (s, 1H), 7 59 (d, 1H), 7.00-7.37 (m, 5H), 6.14 (s, 1H), 5.86 (s, 1H), 3 74 (t, 2H), 3.08 (t, 2H), 2.39 (s, 3H), 2.19 (s, 3H).

### Example 12

### N1,N1-Diethyl-N4-(4-methyl-6-piperidin-1-yl-pyrimidin-2-yl)-pentane-1,4-diamine (CWB008)

The title compound was prepared from piperidine as described in Example 3
¹H NMR (300 MHz, CDCl₃): δ 5.73 (s, 1H), 4.67 (d, 1H), 4.02 (m, 1H), 3.54 (m, 4H), 2.50 (q, 4H), 2.42 (m, 2H), 2.17 (s, 3H), 1.43-1.65 (m, 10H), 18 (d, 3H), 1.00 (t, 6H).

### Example 13

### N,N-Diethyl-N'-[4-methyl-6-(2-methyl-quinolin-6-yloxy)-pyrimidin-2-yl]-propane-1,3-diamine (CWB011)

The title compound was prepared from 6-amino-2-methylquinoline as described in Example 3 3-(diethylamino)-propylamine was used instead of 2-amino-5-diethylaminopentane.
¹H NMR (300 MHz, CDCl₃): δ 8.03-7.99 (m, 2H), 7.55-7.47 (m, 2H), 7.30 (d, 1H), 5.91 (s, 1H), 5.66 (br. s, 1H), 2.75 (s, 3H), 2.48-241 (m, 6H), 2.27 (s, 3H), 1.65-1.60 (m, 4H), 0.96 (t, 6H).

### Example 14

### [2-(4-Diethylamino-1-methyl-butylamino)-6-methyl-pyrimidin-4-ylamino]-acetic acid (CWB015)

[2-(4-Diethylamino-1-methyl-butylamino)-6-methyl-pyrimidin-4-ylamino]-acetic acid ethyl ester (CWB012) (90 mg, 0.256 mmol) was dissolved in dioxane (10 mL) and LiOH (31 mg, 1.23 mmol) in H₂O (5 mL) was added to the solution The reaction mixture was stirred at room temperature for 2.5 h. H₂O was added to the mixture and pH was adjusted to 6 with 1 M HCl. The mixture was extracted with CH₂Cl₂, the organic layer was dried under Na₂SO₄ and then evaporated The crude material was purified with MPLC (medium pressure liquid chromatography) using acetonitril/H₂O as eluent to give 34 mg (41 %) of the title compound.

The compound was made to its hydrochloride salt with 0.3 mmol of 1M HCl in diethylether..
¹H NMR (300 MHz, CD₃OD): δ 5.94 (s, 1H), 4.19-4.14 (m, 1H), 4.07 (d, 1H), 3.70 (d, 1H), 3.20 (q, 4H), 3.06 (q, 2H), 2.24 (s, 3H), 1.78-1.46 (m, 4H), 1.31-1.25 (m, 9H).

### Example 15

### N2,N4-Bis-(1H-indol-5-ylmethyl)-pyrimidine-2,4-diamine(CWB020)

The title compound was prepared from 5-(aminomethyl)-indole and 2,4-dichloropyrimidine as described in Example 3.
¹H NMR (300 MHz, CDCl₃) δ 8.15 (m, 2H), 7 86 (d, 1H), 7.61 (d, 2H), 7.33 (m, 2H), 7.23-7.15 (m, 4H), 6.51 (m, 2H), 5.75 (d, 1H), 5.18 (m, 1H), 4.96 (m, 1H), 4.69 (d, 2H), 4.59 (d, 2H).

### Example 16

### N2-(4-Diethylamino-1-methyl-butyl)-N4-(2-methyl-1H-indol-5-yl)-pyrimidine-2,4-diamine CWB021)

The title compound was prepared from 5-amino-2-methylindole, 2,4-dichloropyrimidine and 2-amino-8-diethylaminopentan as described in Example 3.
¹H NMR (300 MHz, CD₃OD), δ 7.68 (d, 1H), 7.58 (s, 1H), 7.22 (d, 1H), 7.06 (dd, 1H), 6.09 (s, 1H), 5.92 (d, 1H), 4.11-4.08 (m, 1H), 2.67-2.54 (m, 6H), 2 42 (s, 3H), 1.61-1.53 (m, 4H), 1 22 (d, 3H), 1.03 (t, 6H).

### Example 17

### N2-(4-Diethylamino-1-methyl-butyl)-N4-quinolin-3-yl-pyrimidine-2.4-diamine (CWB022)

The title compound was prepared from 3-aminoquinoline, 2,4-dichloropyrimidine and 2-amino-5-diethylaminopentan as described in Example 3.
¹H NMR (300 MHz, CDCl₃). δ 8.92 (d, 1H), 8.74 (s, 1H), 8.17 (br, 1H), 8.00 (m, 2H), 7.74 (dd, 1H), 7.27-7.56 (m, 2H), 5.89 (d, 1H), 5 58 (br, 1H), 4.00 (m, 1H), 2.42-2.57 (m 6H), 1.60 (m, 4H), 1 28 (d, 3H), 1.00 (t, 6H).

### Example 18

### N2-(4-Diethylamino-1-methyl-butyl)-N4-(1H-indol-6-yl)-pyrimidine-2,4-diamine (CWB023)

The title compound was prepared from 6-aminoindole, 2,4-dichloropyrimidine and 2-amino-5-diethylaminopentan as described in Example 3
¹H NMR (300 MHz, CDCl₃), δ 7.86(d, 1H), 7.82 (br, 1H), 7.55 (d, 1H), 7.19 (m, 1H), 6.81 (d, 1H), 6.63 (br, 1H), 6.50 (s, 1H), 5 89 (d, 1H), 4 81 (d, 1H), 3.97 (m, 1H), 2.53-2.71 (m 6H), 1.67-1.96 (m, 4H), 1 18 (d, 3H), 1.04 (t, 6H).

### Example 19

### N2-(4-Diethylamino-1-methyl-butyl)-N4-(1H-indol-5-yl)-pyrimidine-2,4-diamine (CWB024)

The title compound was prepared from 5-aminoindole, 2,4-dichloropyrimidine and 2-amino-5-diethylaminopentan as described in Example 3
¹H NMR (300 MHz, CDCl₃): δ 8.84 (br, 1H), 7.83(d, 1H), 7.56 (d, 1H), 7.36 (d, 1H), 7.24 (m, 1H), 7.08 (dd, 1H), 6.96 (br, 1H), 6.51 (s, 1H), 5.88 (d, 1H). 4.68 (d, 1H), 4.03 (m, 1H), 3.11 (br, 1H), 2.56 (q, 4H), 2.45 (m 2H), 1.42-1 52 (m, 4H), 1 15 (d, 3H), 1.03 (t, 6H).

### Example 20

### 3-{2-[2-(4-Diethylamino-butylamino)-6-methyl-pyrimidin-4-ylamino]-ethyl}-1H-indol-5-ol (CWB026)

The title compound was prepared from serotonin hydrochloride, 2,4-dichloro-6-methylpyrimidine and 3-(diethylamino)-propylamine as described in Example 3.
¹H NMR (300 MHz, CDCl₃): δ 8.69 (br, 1H), 7.10(d, 1H), 6.95 (s, 1H), 6.84 (s, 1H), 6.74 (d, 1H), 5.42 (br + s, 2H), 5.00 (m, 1H), 3.48 (m, 2H), 3.36 (m, 2H), 2.83 (m, 2H), 2.55 (m, 6H), 2 08 (s, 3H), 1.70 (m, 2H), 0.95(t, 6H)

### Docking experiments

In order to analyse the Rac1 inhibitory effect of the compounds of the invention, a docking analysis has been performed utilizing the crystal structure of Rac1.

A docking analysis includes the analysis of the orientation of one molecule with respect to a second molecule to which it is bound forming a complex.

Table 1 below summarizes the protein-protein interface GlideScore for all the compounds of the invention.

The GlideScore, in particular, provides a measure of the binding affinity (ΔGb) between Rac1 and a ligand/compound of the invention. It measures the electrostatic interactions between molecules, including polar-polar contacts, hydrogen bonds and ionic bridges, as well as van der Waals interactions, torsional and desolvation energies, in addition to steric clashes. The efficiency measure of ΔGb/P.S.A was also developed and computed, wherein P.S.A is the polar surface area in Angstromm² of a particular ligand. The deeper the negative GlideScore/P.S.A values are, the stronger is the expected binding affinity of the different molecules to Rac1. The results obtained for the referenced compound NSC23766 are also included.

**Table 1: Docking results of compounds with Rac1 inhibitory activity (software Glide).**

| **Compound** | **AGb** | Δ**Gb/P.S.A.** | | **Compound** | Δ**Gb** | Δ**Gb/P.S.A.** |
|---|---|---|---|---|---|---|
| NSC23766 | -9.16 | -0.1753 | | CWB014/01032 | -5.93 | -0.1139 |
| CWB001/01018 | -9.58 | -0.1558 | | CWB015/01031 | -7.85 | -0.0987 |
| CWB002/02038 | -9.87 | -0.1789 | | CWB016/02046 | -8.93 | -0.1436 |
| CWB003/01020 | -9.18 | -0.1757 | | CWB017/02047 | -8.58 | -0.162 |
| CWB004/01022 | -8.78 | -0.168 | | CWB018/01034 | -6.37 | -0.0981 |
| CWB005/02039 | -10.08 | -0.1391 | | CWB019/01037 | -7.19 | -0.1823 |
| CWB006/02035 | -10.43 | -0.1396 | | CWB020/01041 | -10.25 | -0.1788 |
| CWB007/02040 | -8.42 | -0.1354 | | CWB021/01039 | -9.14 | -0.1986 |
| CWB008/02044 | -8.34 | -0.1575 | | CWB022/02052 | -6.93 | -0.1607 |
| CWB009/01017 | -7.07 | -0.1476 | | CWB023/02053 | -7.93 | -0.1724 |
| CWB010/01024 | -6.71 | -0.1288 | | CWB024/02054 | -7.77 | -0.1689 |
| CWB011/01023 | -8.33 | -0.1355 | | CWB025/02056 | -8.54 | -0.129 |
| CWB012/01025 | -8.11 | -0.1235 | | CWB026/02057 | -9.03 | -0.1363 |
| CWB013/01028 | -6.37 | -0.0966 | | CWB027/02058 | -7.87 | -0.1188 |

As from the results shown in Table 1, compared to the NSC23766 which has a ΔGb/P.S.A ratio equal to -0.1753, compounds CWB002, 003, 019, 020, 021, 023, and 024 have similar ΔGb/P.S.A value and are expected to elicit a significant inhibitory action on Rac1 activity.

The structure of the docked ligands occupied the same binding site as NSC23766 on the Rac1 surface.

### Analysis of Rac1 inhibitory activity

To determine the effect of the compounds of the invention on Rac1 activity we have utilized human cultured cells. The cells have been incubated for 4 hours with a fixed concentration of each invention compound and then Rac1 activity has been stimulated by adding 10 ng/ml of PDGF-BB to the culture medium. The intracellular amount of Rac1-GTP has been determined using the GLISA™ assay (Cytoskeleton, Inc). The GLISA™ assay uses a 96-well plate coated with RBD domain of Rac1-family effector protein PAK1. The active GTP-bound form of Rac1, but not the inactive GDP-bound form from a total cell lysates of human cells binds to the plate. Accordingly, bound active Rac-1 protein was then detected by incubation with a specific primary antibody followed by a secondary antibody conjugated to horseradish peroxidase (HRP) The signal is then developed with O-phenylenediaminereagent (OPD reagents). A significant inhibition on Rac1 activity should decrease the intracellular levels of Rac1-GTP and therefore reduce the percentage of Rac1-GTP detected compared to control untreated cells.

**Table 2 below shows the Rac1 inhibitory activity of the tested compounds measured in human smooth muscle cells by G-LISA assay on a double two experiments base**

| **Compound** | **Concentration (µm)** | **Rac1 inhibitory activity (% vs control)** |
|---|---|---|
| Control PDGF-BB 10ng/ml | | 100 |
| NSC23766 | 50 | 89.9 |
| CWB001/01018 | 50 | 102.5 |
| CWB002/02038 | 50 | 109.4 |
| CWB003/01020 | 50 | 93.8 |
| CWB004/01022 | 50 | 109.7 |
| CWB005/02039 | 50 | 105.8 |
| CWB006/02035 | 50 | 91.5 |
| CWB007/02040 | 50 | 100.7 |
| CWB008/02044 | 50 | 95,8 |
| CWB009/01017 | 50 | 103.2 |
| CWB010/01024 | 50 | 124.6 |
| CWB011/01023 | 50 | 94.8 |
| CWB012/01025 | 50 | 92.9 |
| CWB013/01028 | 50 | 100.1 |
| CWB014/01032 | 50 | 106.7 |
| CWB015/01031 | 50 | 83.9 |
| CWB016/02046 | 50 | 93.2 |
| CWB017/02047 | 50 | 92.8 |
| **CWB018/01034** | 50 | **34.2** |
| CWB019/01037 | 50 | 101.3 |
| **CWB020/01041** | 50 | **17.1** |
| **CWB021/01039** | 50 | **81.1** |
| **CWB022/02052** | 50 | **77.6** |
| CWB023/02053 | 100 | 98.0 |
| **CWB024/02054** | 50 | **58.3** |
| CWB025/02056 | 100 | 122.8 |
| CWB026/02057 | 100 | 107.9 |
| CWB027/02058 | 100 | 110.7 |

The bolded compounds have a significant higher efficiency than the reference compound NSC23766

Thus, compound CWB018, 020, 021, 022, and 024 showed a significant end efficient inhibitory action on Rac1 activity and are selected for further investigation Under our experimental conditions we observed only an 11% inhibitory effect of NSC23766 on Rac1 activity compared to 18 9÷82.3% inhibition of the compounds of the invention Accordingly, compounds CWB018/01034, CWB020101041, CWB021/01039, CWB022/02052, and CWB024/02054, which show the highest inhibitory activity, have been selected for further analysis. Thus, a classic pull down assay was also performed in human cells incubated with 50 and 100 µM concentration of CWB024/02054

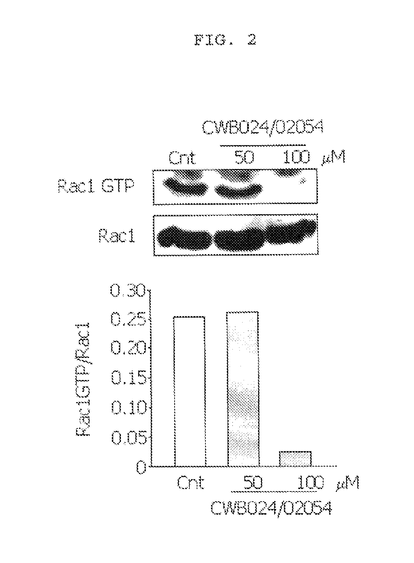

As shown in Figure 2, CWB024/02054 very efficiently reduced the intracellular content of Rac1-GTP by over 90%.

### Anti-apoptotic effect of selected compounds on ischemic cardiac myocytes in culture

To establish a cardiomyocytes based apoptosis assay, which mimics the post-ischemic damage observed after myocardial infarction, we have utilized a cardiac muscle cell line, HL-1 cells, derived from the AT-1 mouse atrial cardiomyocyte tumor lineage. The HL-1 cells were incubated in glucose deficient medium in the presence of hydrogen peroxide and either in presence or absence of compounds of the invention Apoptosis rate of ischemic HL-1 cells was normalised to the apoptosis rate of the 8h control cells under non-ischemic conditions, thus incubated with normal concentration of glucose in the absence of hydrogen peroxide. Both non-ischemic and ischemic HL-1 cultures were harvested after 8 hours. Analyzed concentration of NSC23766 and the selected compounds with Rac1 inhibitory activity, CWB021/01039, CWB022/02052, and CWB024/02054 was 100 µM. The apoptotic rates were then evaluated by TUNEL assay, a method for detecting DNA strand breaks in apoptotic cells by flow cytometer The solvent of the compounds, i.e DMSO, has no influence on the apoptosis rate. As shown in Table 3 below, apoptosis is increased in ischemic cultures compared with the corresponding control culture NSC23766 and the compounds of the invention reduced the apoptotic rate in ischemic cultures compared to the 8 h ischemia

**Table 3. Effect of newly synthesized compounds with Rac1 inhibitory activity on apoptosis rate in HL-1 cells normalized to control (TUNEL-assay) The data are representative of two experiments.**

| | **Relative rate of apoptosis normalised to control [%]** |
|---|---|
| 8h Control | 100 |
| DMSO Control | 94.6 |
| 8h Ischemia | 701.6 |
| Ischemia + NSC23766 | 546.5 |
| Ischemia + CWB021/01039 | 511.6 |
| Ischemia + CWB022/02052 | 607.8 |
| Ischemia + CWB024/02054 | 319.4 |

From the above data, the compound CWB024/02054 showed to have the best inhibitory effect with approximately 50% reduction of relative apoptosis.

### Toxicity profile of selected compounds with Rac1 inhibitory activity

To measure the cytotoxic potential of the compounds of the invention the so called resazurine assay was applied which investigates the metabolic activity as biological endpoint Compounds CWB018/01034, CWB020/01041, CWB021/01039, CWB022/02052, and CWB024/02054 were prepared as 100 mM stock solutions in acetonitrile and incubated with the cultured cells at 8 different concentrations starting with 1 mM and followed by 1:2 dilution steps (1 mM, 500 µM, 250 µM, 125 µM, 62.5 µM, 31 25 µM, 15.625 µM, and 7.8125 µM). For a pre-screening of the compounds they were first tested in human hepatocellular carcinoma cells (HepG2 cells)for 24 h and to gain further information about the cytotoxic potential more specific target tissues like the mouse cardiomyocytes HL-1 and primary rat cardiomyocytes were used additionally. In Table 4 below the calculated EC₅₀ values (dose with half maximal effect) determined from the dose-response curves, maximal viability concentration (MVC) pointing out the first concentration with clear reduction of metabolic activity and classification are indicated A classification scheme was previously set up by screening known reference compounds and calculation of their EC₅₀ values Accordingly, the compounds can be ranked into high toxic (EC₅₀ ≤ 10 µM), medium toxic (10 µM < EC₅₀ ≤ 100 µM), low toxic (100 µM < EC₅₀ ≤ 500 µM) and not toxic (EC₅₀ > 500 µM). Compound NSC23766 which was only tested in HepG2 can be classified as not toxic since an EC₅₀ determination was not possible due to low toxic effects up to the maximal test concentration whereas compound CWB018/01034 has a low toxic potential in HepG2 cells and all other compounds can be classified as medium toxic having EC₅₀ values ranging from 21 µM to 64 µM In HL-1 and primary rat cardiomyocytes all the compounds of the invention can be classified as medium toxic Compound CWB018/01034 had a higher toxic potential in HI-1 and primary rat cardiomyocytes compared to HepG2, all other compounds reached the same toxic classification in the three tested cell types

**Table 4 shows the summary of the cytotoxicity profile and classification for compounds of the invention with Rac1 inhibitory activity The EC₅₀, MVC determined for the endpoint metabolic activity in HepG2, HL-1 and primary rat cardiomyocytes after 24 h of compound exposure and the consequent classification is displayed (the data are representative of two repeated experiments).**

| | | | | | | | | | |
|---|---|---|---|---|---|---|---|---|---|
| | **HepG2** | | | **HL-1** | | | **Primary Rat Cardiomyocytes** | | |
| | | EC₅₀ (µM) | MVC (µM) | | EC₅₀ (µM) | MVC (µM) | | EC₅₀ (µM) | MVC (µM) |
| NSC23766 | no | > 500 | 31.2 | - | - | - | - | - | - |
| CWB018/01034 | + | 162 | 62.5 | ++ | 68 | 15.6 | ++ | 85 | 31.2 |
| CWB020/01041 | ++ | 64 | 7.8 | ++ | 39 | 15.6 | ++ | 17 | 3.9 |
| CWB021/01039 | ++ | 21 | 7.8 | ++ | 24 | 31.2 | ++ | 13 | 31.3 |
| CWB022/02052 | ++ | 32 | 7.8 | ++ | 60 | 31.2 | ++ | 15 | 7.8 |
| CWB024/02054 | ++ | 33 | 7.8 | ++ | 56 | 7.8 | ++ | 17 | 1.9 |

| | | | | | | | | | |
|---|---|---|---|---|---|---|---|---|---|
| Cytotoxicity-Classification: EC50 ≤ 10 µM, high (+++) 10 µM < EC50 ≤ 100 µM, medium (++) 100 µM < EC50 ≤ 500, low (+) EC50 > 500 µM, (no) | | | | | | | | | |

The toxicity classification has been determined by using drugs currently utilized in clinic with known toxicity profile

### Analysis of permeation properties of selected compounds with Rac1 inhibitory activity

The permeation characteristics of the Rac1 inhibitors were determined first in the artificial system PAMPA (Parallel Artificial Membrane Permeability Assay) which uses a membrane covered with a lipid to simulate the lipid bilayer of various cell types, including intestinal epithelium and second *in vitro* across Caco2, a human colon carcinoma cell line seeded as monolayers onto polycarbonate membranes. The PAMPA is able to predict the ability of a compound to permeate by a passive transcellular transport through an artificial lipid layer assembled on a membrane and also allows a classification into low (flux <20%), medium (20%≤ flux ≤70%), and high (flux >70%) absorbing substances. The Caco2 assay provides a well-established *in vitro* model to predict the human intestinal absorption. In their differentiated phase, Caco2 cells exhibit the morphological and physiological properties of the human small intestine, e.g. barrier function, active transport systems and efflux systems. According to the compounds apparent permeation, i.e Papp values which are expressed as *10⁻⁶ cm/s, in Caco2 cells, they can be ranked into 3 categories:
- compounds with a Papp <1 is predicted to be poorly absorbed (0 - 20% in vivo human absorption);
- compounds with Papp between 1 and 10 are moderately absorbed (20 - 70%); and
- compounds with a Papp >10 are predicted to be well absorbed (70 - 100%) Both assays are important in the early drug discovery, as they may help predicting oral absorption, a drug's most prominent way of entry into the blood stream and a key success factor in the drug development process

The flux rate of the compounds of the invention and the reference NSC23766 was measured from a donor compartment to an acceptor compartment across the lipid layer at a start concentration of 250 µM over a diffusion period of 16 h. According to the PAMPA-classification scheme, the tested compounds can be ranked as follows.
- low permeable compounds; NSC23766, CWB021/01039 and CWB024/02054;
- medium permeable is CWB022/02052, and
- high permeable compounds are CWB018/01034 and CWB020/01041.

The calculations of the apparent permeation across Caco2 monolayers over a period of 90 min with a start concentration of 50 µM resulted in the classification of the tested compounds as follows.
- medium penetration was detected for NSC23766 and CWB022/02052
- high permeable are CWB018/01034, CWB020/01041, CWB021/01039 and CWB024/02054

For those compounds with a low diffusion rate across the artificial membrane in the PAMPA but a medium or high permeability across Caco2 layers, i.e. NSC23766, CWB021/01039 and CWB024/02054, an active transport mechanism is assumed Thus, compounds CWB018/01034, CWB020/01041, CWB021/01039 and CWB024/02054 show better permeation characteristics compared to the reference NSC23766 Results are depicted in table 5.

**Table 5 shows PAMPA flux values, Papp-values of the invention compounds with Rac1 inhibitory activity, and their classification.**

| | PAMPA flux [%] ± SD | PAMPA classification | Pₐₚₚ [x10-6cm/s]±SD | Caco2 Classification |
|---|---|---|---|---|
| NSC23766 | 11±4 | low | 2.4±4.2 | medium |
| CWB018/01034 | 85±11 | high | 20.5±1.9 | high |
| CWB020/01041 | 54±4 | high | 65.9±3.1 | high |
| CWB021/01039 | 5±1 | low | 17.1±12.5 | high |
| CWB022/02052 | 34±4 | medium | 8.9±2.7 | medium |
| CWB024/02054 | 6±2 | low | 32.1±12.4 | high |

### Determination of metabolic stability of selected compounds with Rac1 inhibitory activity

The determination of the metabolic stability of compounds of the invention with Rac1 inhibitory activity were carried out by incubating the tested compounds with liver homogenates in the presence of appropriate cofactors

The basic incubation mixture with total volume of 500 µl consisted of the following components. 100 µl liver homogenate, compound in DMSO (dimethyl sulfoxide) and 1 mM NADPH (reduced nicotinamide adenine dinucleotide phosphate), 1 mM UDPGA (uridine 5'-diphosphoglucuronic acid), 1 mM PAPS (3'-phosphoadenosine-5'-phosphosulfate) and 1 mM GSH (glutathione). The substrate concentration used was 10 µM. Two parallel incubations, one with cofactors and one without, were made. Final DMSO concentration in the incubation was 1 µM. Each reaction mixture was preincubated for 2 minutes at +37°C in a shaking incubator block (Eppendorf Thermomixer 5436, Hamburg, Germany). Reaction was started by addition of a cofactor mixture and stopped by adding 250 µl of ice-cold acetonitrile.

The remaining LC/MS peak areas (relative to 0 min incubation samples, %) detected for the substrate were as follows:
CWB18/01034 disappear completely in the 60 minute incubation time, the disappearance being completely non-cofactor dependent

For CWB022/02052 5% of LC/MS peak area was remaining after 60 minute incubation, the disappearance being mainly cofactor dependent and thus dominated by CYP-related metabolism

For CWB020/01041 and CWB021/01039, about 33 - 41% LC/MS peak area was remaining after 60 min incubation, the detected disappearance being mainly cofactor dependent, even thought some disappearance was detected also without cofactors for some of the compounds. CWB024/02054 was the most stable of the substances in the incubations, having about 91% remaining peak area after 60 minute incubation and disappearance is clearly non-cofactor dependent

The remaining peak areas after 60 minutes incubation was also determined after incubation with increasing concentrations of compounds CWB021/01039 and CWB024/02054. The remaining peak areas for CWB021/01039 with 10 µM, 1 µM and 0.1 µM initial substrate concentrations were 95%, 81 % and 69%, respectively, while the corresponding values for CWB024/02054 were 86%, 87% and 76%, respectively. The disappearances for both study compounds were also clearly cofactor dependent and thus metabolism related Calculations of pharmacokinetic (PK) parameters on the basis of substrate depletion for both study compounds (1 µM, 60 min time point) in human liver microsomes are shown in Table 6.

**Table 6. Kinetic in vitro - in vivo extrapolations of study compound clearances and half-lifes in human, wherein: intrinsic clearance (whole liver) = CLᵢₙₜ; hepatic clearance = CL_{H}; total (systemic) clearance = CLₜₒₜₐₗ; half-life = t_{1/2}.**

| **Substance** | CLᵢₙₜ(L/min) | CLₕ(L/h) | CLₜₒₜ(L/h) | t_{1/2}(h) |
|---|---|---|---|---|
| CWB0021 | 0.31 | 15.14 | 15.32 | 7.3 |
| CWB0024 | 0.2 | 10.63 | 10.81 | 10.34 |

All the detected metabolites were tentatively identified according to the accurate mass data and in-source fragment ion data Accordingly, the main metabolic reactions for all the compounds were hydroxylations and dealkylations and their combinations.

The relative percentage amount of each metabolite from total combined metabolite LC/MS peak areas for the CWB18/01034, CWB020/01041, CWB021/01039, CWB022/02052, and CWB024/02054 are shown in the Table 7 below

**Table 7. LC/ESI/TOF-MS data obtained for the substrate and its metabolites The % means the relative share of the total combined metabolite peak area for the particular compound.**

| **Compound** | **RT [min]** | **Identification** | **meas.(m/z) [M+H]⁺** | **calc.(m/z)** | **%** |
|---|---|---|---|---|---|
| **CWB018** | 3.95 | Parent | 380.2049 | 380.2087 | |
| **M1** | 3.79 | Demethylation | 366.1908 | 366.1930 | 98.5 |
| **M2** | 3.74 | Demethylation + Hydroxylation | 382.1874 | 382.1879 | 0.6 |
| **M3** | 4.03 | Demethylation + decarbossilazione | 322.2084 | 322.2032 | 0.5 |
| **M4** | 3.20 | N-dealkylation (loss of methyl + methylpentanoate) | 252.1230 | 252.1249 | 0.5 |
| **CWB020** | 4.03 | Parent | 369.1803 | 369.1828 | |
| **M1** | 3.85 | Hydroxylation | 385.1804 | 385.1777 | 75.0 |
| **M2** | 3.79 | 2× Hydroxylation | 401.1762 | 401.1726 | 13.6 |
| **M3** | 3.92 | Hydroxylation + Sulphate conjugation | 465.1374 | 465.1345 | 11.4 |
| **CWB021** | 3.68 | Parent | 381.2791 | 381.2767 | |
| **M1** | 3.63 | Deethylation | 353.2480 | 353.2454 | 100 |
| **CWB022** | 3.61 | Parent | 379.2646 | 379.2610 | |
| **M1** | 3.61 | Deethylation | 351.2313 | 351.2297 | 6.3 |
| **M2** | 3.47 | Hydroxylation | 395.2572 | 395.2559 | 8.0 |
| **M3** | 3.60 | Hydroxylation | 395.2560 | 395.2559 | 61.8 |
| **M4** | 3.63 | 2 × Hydroxylation | 411.2525 | 411.2508 | 7.0 |
| **M5** | 3.58 | Deethylation + Hydroxylation | 367.2274 | 367.2246 | 16.9 |
| **CWB024** | 3.63 | Parent | 367.2627 | 367.2610 | |
| **M1** | 3.58 | Deethylation | 339.2326 | 339.2297 | 60.0 |
| **M2** | 3.37 | Hydroxylation | 383.2607 | 383.2559 | 36.0 |
| **M3** | 3.21 | 2× Hydroxylation | 399.2553 | 399.2508 | 4.0 |

### Inhibition towards CYP enzymes

The effects of 10 µM of the invention compounds on major drug metabolising Cytochrome P450 (CYP) activities were measured Neither of the invention compounds did notably inhibit any of the model activities studied, and consequently, the IC₅₀ values of both CWB021/01039 and CWB024/02054 against major drug metabolising CYPs were above 10 µM. The activity affected most with both study compounds was associated with CYP2D6, but was still inhibited only by 35% and 30% by 10 µM CWB021/01039 and CWB024/02054, respectively. Thus, the likelihood that CWB021/01039 and CWB024/02054 could cause major drug-drug interactions *in vivo* is relatively low.

## Claims

1. A compound of general formula (I): 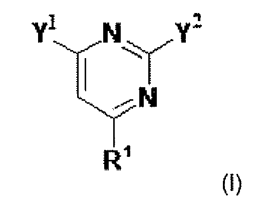 wherein
Y¹ may be
1) -NR²R³, wherein R² is H and R³ may be
a) a saturated or unsaturated straight or branched C1-C18 carbon chain, optionally interrupted by one or more heteroatoms and being optionally substituted with one or more carboxylic acid (-COOH) or carboxylic acid ester (-COOR₈) group or hydroxyl group (-OH) or thiol group (-SH) or halogen or amino group (-NH₂) or substituted amino group (-NR⁸₂) or nitro group (-NO₂) or with aromatic group Ar having from 9 to 10 ring carbons, wherein from 1 to 4 carbon atoms may be substituted with a heteroatom selected from -N-, -O- and -S-, said Ar group being optionally substituted at any available position with a saturated or unsaturated straight or branched C1-C4 carbon chain or a straight or branched C1-C6 alkoxy group or C3-C6 cycloalkyl group or a hydroxy group (-OH) or a thiol group (-SH) or an halogen or an amino group (-NH₂) or a substituted amino group (-NR⁸₂) or a nitro group (-NO₂), wherein R⁸ is a C1-C8 straight or branched alkyl group; or
b) R⁴, wherein R⁴ is an aromatic group Ar as above disclosed; or wherein
c) R² and R³, together with the N atom they are attached to, form a 5 or 6 membered heterocycle, wherein from 1 to 3 carbon atoms may be substituted with an heteroatom selected from -N-, -O- or -S-, said heterocycle being optionally substituted at any available position with a saturated or unsaturated straight or branched C1-C4 carbon chain or a C3-C6 cycloalkyl group or a straight or branched C1-C6 alkoxy group or with one or more carboxylic acid (-COOH) or carboxylic acid ester (-COOR⁸) group or a hydroxy group (-OH) or a thiol group (-SH) or an halogen or an amino group (-NH₂) or a substituted amino group(-NR⁸₂), wherein R⁸ is as above disclosed, or a nitro group (-NO₂);or wherein Y1 may be
2) -O-R³, wherein R³ is as above disclosed; and wherein
Y² is -N-R⁵R⁶, wherein
d) R⁵ is H and R⁶ is R³ or R⁴, wherein R³ and R⁴ are as above disclosed; or wherein
e) R⁵ and R⁶ together with the N atom they are attached to form a 5 or 6 membered heterocycle, wherein from 1 to 3 carbon atoms may be substituted with an heteroatom selected from -N-, -O- or -S-, said heterocycle being optionally substituted at any available position with a saturated or unsaturated straight or branched C1-C4 carbon chain or a C3-C6 cycloalkyl group or a straight or branched C1-C6 alkoxy group or with one or more carboxylic acid (-COOH) or carboxylic acid ester (-COOR⁸) group or a hydroxy group (-OH) or a thiol group (-SH) or an halogen or an amino group (-NH₂) or a substituted amino group(-NR⁸₂), wherein R⁸ is as above disclosed, or a nitro group (-NO₂), and wherein
R¹ is H or a saturated or unsaturated straight or branched C1-C18 carbon chain; or a pharmaceutically acceptable salts thereof

2. The compounds of claim 1, wherein
a. when Y¹ is NR²R³, R² is H and R³ is
i. a saturated straight or branched C1-C7 carbon chain optionally substituted with one or more carboxylic acid (-COOH) group or carboxylic acid ester (-COOR⁸) group or a substituted amino group (-NR⁸₂) or with an aromatic group Ar having from 9 to 10 ring carbons, wherein one atom is replaced with nitrogen, said Ar group being optionally substituted at any available position with one or more an hydroxyl (-OH) group or methyl group (-CH₃), wherein R⁸ is a C1-C4 saturated straight or branched carbon chain; or
ii. R³ is R⁴, wherein R⁴ is an Ar group having from 9 to 10 members, wherein one carbon atom is replaced with nitrogen, said Ar group being optionally substituted at any available position with one or more hydroxy (-OH) group or methyl group (-CH₃); or
iii. R² and R³ together with the nitrogen atom they are attached to form a 6 membered heterocycle optionally comprising an oxygen atom,
or wherein
b Y¹ may be -O-R⁴, wherein R⁴ is an aromatic group Ar having 10 ring carbons, wherein optionally one carbon atom may be substituted with nitrogen, said Ar group being optionally substituted at any available position with a methyl group (-CH₃);
and wherein Y² is -NR⁵R⁶, wherein
d) R⁵ is H and R⁶ is a saturated straight or branched C1-C7 carbon chain optionally substituted with a substituted amino group (-NR⁸₂) or with an aromatic group Ar having 9 ring carbons, wherein one carbon atom is replaced with nitrogen, said Ar group being optionally substituted at any available position with one or more (an) hydroxyl (-OH) group and wherein R⁸ is as above disclosed, or wherein
e) R⁵ and R⁶ together with the nitrogen atom they are attached to form a 6 membered heterocycle, wherein from 1 to 2 carbon atoms are substituted with nitrogen or oxygen, and wherein R1 is H or C1-C4 straight or branched saturated carbon chain, or a pharmaceutically acceptable salts thereof.

3. The compounds of claims 1 or 2, wherein when Y¹ is NR²R³ and R² is H, R³ is
i. -(CH)(COOCH₃)(CH₂-CH-(CH₃)₂) or -CH(CH₃)(CH₂)₃-N(CH₂CH₃)₂ or -CH₂-COOH or -CH₂-COOCH₂CH₃, or a - CH₂- or a -CH₂-CH₂- group substituted with indole substituted with a hydroxyl (-OH) or methyl (-CH₃) group;
ii quinoline or isoquinoline or indole, optionally substituted with a hydroxyl (-OH) or methyl (-CH₃)group or both; or
iii R² and R³ together with the nitrogen atom they are attached to form a piperidine or a morpholine cycle,
or when Y¹ is -O-R³, wherein R³ is quinoline or isoquinoline or naphthalene, optionally substituted with a hydroxyl (-OH) or methyl (-CH₃) group;
and (wherein) when Y² is -NR⁵R⁶, wherein R⁵ is H and R⁶ may be
iv. -CH(CH₃)(CH₂)₃-N(CH₂CH₃)₂ or -CH-(CH₂)₃-N(CH₂CH₃)₂ or - CH-(CH₂)₄-N(CH₂CH₃)₂ or a -CH₂- or a -CH₂-CH₂- group substituted with indole substituted with a hydroxyl (-OH) or methyl (-CH₃) group or R⁶ may be
quinoline or isoquinoline, optionally substituted with a hydroxyl (-OH) or methyl (-CH₃) group or both; or
v. R⁵ and R⁶ together with the nitrogen atom they are attached to form a piperidine or morpholine ring;
and wherein R¹ is H or methyl;
or a pharmaceutically acceptable salts thereof

4. A compound according to claims 1 to 3, which is selected from:
N2-(4-Diethylamino-1-methyl-butyl)-N4-quinolin-6-yl-pyrimidine-2,4-diamine;
N1,N1-Diethyl-N4-(4-methyl-6-morpholin-4-yl-pyrimidin-2-yl)-pentane-1,4-diamine;
N1,N1-Diethyl-N4-(6-methyl-2-mopholin-4-yl-pyrimidin-4-yl)-pentane-1,4-diamine;
[2-(4-Diethylamino-1-methyl-butylamino)-6-methyl-pyrimidin-4-ylamino]-acetic acid ethyl ester;
4-Methyl-2-[6-methyl-2-(qurinolin-6-ylamino)-pyrimidin-4-ylamino]-pentanoic acid methyl ester,
N1,N1-Diethyl-N4-[4-naphthalen-2-yloxy)-pyrimidin-2-yl]-pentane-1,4-diamine;
3-{2-[2-(4-Diethylamino-1-methyl-butylamino)-6-methyl-pyrimidin-4-ylamino]-ethyl}-1H-indol-5-ol,
3-{2-[4-(4-diethylamino-1-methyl-butylamino)-6-methyl-pyrimidin-2-ylamino]-ethyl}-1H-indol-5-ol;
N1,N1-Diethyl-N4-[4-methyl-6-(2-methy)-quinolin-6-yloxy)-pyrimidin-2-yl]-pentane-1,4-diamine;
N2-(4-Diethylamino-1-methyl-butyl)-N4-[2-(1H-indol-3-yl)-ethyl)-6-methylpyrimidine-2,4-diamine;
N2-(4-Diethylamino-1-methyl-butyl)-6-methyl-N4-quinolin-6-yl-pyrimidine-2,4-diamine;
6-[2-(4-Diethylamino-1-methyl-botylamino)-6-methyl-pyrimidin-4-ylamino]-2-methyl-quinolin-4-ol,
6-{2-[2-(1H-Indol-3-yl)-ethylamino]-6-methyl-pyrimidin-4-ylamino}-2-methyl-quinolin-4-ol;
N1,N1-Diethyl-N4-(4-methyl-6-piperidin-1-yl-pyrimidin-2-yl)-pentane-1,4-diamine;
N,N-Diethyl-N'-[4-methyl-6-(2-methyl-quinolin-6-yloxy)-pyrimidin-2-yl]-propane-1,3-diamine;
[2-(4-Diethylamino-1-methyl-butylamino)-6-methyl-pyrimidin-4-ylamino]-acetic acid;
N2,N4-Bis-(1H-indol-5-ylmethyl)-pyrimidine-2,4-diamine
N2-(4-Diethylamino-1-methyl-butyl)-N4-(2-methyl-1H-indol-5-yl)-pyrimidine-2,4-diamine,
N2-(4-Diethylamino-1-methyl-butyl)-N4-quinolin-3-yl-pyrimidine-2,4-diamine; N2-(4-Diethylamino-1-methyl-butyl)-N4-(1H-indol-6-yl)-pyrimidine-2,4-diamine;
N2-(4-Diethylamino-1-methyl-butyl)-N4-(1H-indol-5-yl)-pyrimidine-2,4-diamine;
3-{2-[2-(4-Diethylamino-butylamino)-6-methyl-pyrimidin-4-ylamino]-ethyl}-1H-indol-5-ol;
or a pharmaceutically acceptable salts thereof

5. A compound according to any one of the preceding claims, which is 4-Methyl-2-[6-methyl-2-(quinolin-6-ylamino)-pyrimidin-4-ylamino]-pentanoic acid methyl ester or N2,N4-Bis-(1H-indol-5-ylmethyl)-pyrimidine-2,4-diamine or N2-(4-Diethylamino-1-methyl-butyl)-N4-(2-methyl-1H-indol-5-yl)-pyrimidine-2,4-diamine or N2-(4-Diethylamino-1-methyl-butyl)-N4-guinolin-3-yl-pyrimidine-2,4-diamine or N2-(4-Diethylamino-1-methyl-butyl)-N4-(1H-indol-5-yl)-pyrimidine-2,4-diamine; or a pharmaceutically acceptable salts thereof.

6. A compound according to any one of the preceding claims, which is N2-(4-Diethylamino-1-methyl-butyl)-N4-(1H-indol-5-yl)-pyrimidine-2,4-diamine or a pharmaceutically acceptable salt thereof

7. A compound according to any one of the preceding claims 1 as a medicament

8. A compound according to claim 7 as a medicament for the treatment or the prevention of the human heart failure, cancers, hypertension, inflammation, and atherosclerosis

9. A pharmaceutical composition comprising one or more compounds of any one of claims 1 to 6 and pharmaceutical additives and/or excipients.

10. The pharmaceutical composition according to claim 9, wherein said additives and/or excipients are selected in the group comprising diluent, solvents, bulking agents, fillers, reological modifier, stabilizers, binders, lubricants, disintegrant, preservatives, pH adjusting agents, buffers, antioxidant, chelating agents, plasticizer, polymers, emulsifiers, edulcorants, flavoring agents, alone or in combination

11. The pharmaceutical composition according to claim 9 or 10 comprising one or more of 4-Methyl-2-[6-methyl-2-(quinolin-6-ylamino)-pyrimidin-4-ylamino]-pentanoic acid methyl ester or N2,N4-Bis-(1H-indol-5-ylmethyl)-pyrimidine-2,4-diamine or N2-(4-Diethylamino-1-methyl-butyl)-N4-(2-methyl-1H-indo)-5-yl)-pyrimidine-2,4-diamine or N2-(4-Diethylamho-1-methyl-butyl)-N4-quinolin-3-yl-pyrimidine-2,4-diamine or N2-(4-Diethylamino-1-methyl-butyl)-N4-(1H-indol-5-yl)-pyrimidine-2,4-diamine, or a pharmaceutically acceptable salts thereof
